# EUROPEAN PATENT SPECIFICATION

(11) **EP 3 061 393 B1**
(45) Date of publication and mention of the grant of the patent: **03.04.2019**
(21) Application number: 15156953.0
(22) Date of filing: 27.02.2015
(51) Int. Cl.: A61B 5/0215

(54) **Implantable pressure sensor device**
Implantierbare Drucksensorvorrichtung
Capteur de pression implantable

(43) Date of publication of application: 31.08.2016
(73) Proprietor: BIOTRONIK SE & Co. KG, 12359 Berlin (DE)
(72) Inventor: Philipp, Jens, deceased (DE); Elsner, Joachim, 14059 Berlin (DE); Biela, Sarah, 10247 Berlin (DE); Pfenniger, Alois, 2502 Biel (CH); Bitzer, Andreas, 8032 Zürich (CH); Ebert, Henning, 10317 Berlin (DE); Skerl, Olaf, 18209 Bad Doberan (DE); van Ooyen, André, 10717 Berlin (DE)
(74) Representative: Galander, Marcus

(56) References cited:
- EP-A1- 1 216 653
- DE-A1-102011 054 732
- US-A- 4 407 296
- US-B1- 8 209 031

## Description

The invention relates to an implantable pressure sensor device, in particular to a miniature pressure sensor device which is implantable in an animal or human body.

Pressure sensors devices including micro electromechanical system (MEMS) chips are known in the art. Pressure sensor devices with MEMS chips are used, e.g., as an implantable cardiac device which may be implanted in a patient to improve the function of the patient's heart. For precise pressure measurements with an accuracy of 2 mbar or less MEMS chips are known having dimensions of, e.g., < 1 mm · 1mm · 0,3 mm.

For usage in a reactive medium such as blood, MEMS chips have to be protected against the reactive medium and are usually embedded in an inert liquid and hermetically sealed against the reactive medium. Typical constructions employ metallic housings which provide a window covered with a pressure transmitting membrane. For the purpose of a medical implant titanium is usually chosen because of its biocompatibility and long-time stability. The MEMS chip is arranged in an incompressible and inert liquid, such as oil, inside the housing. A typical thickness of the housing is more than 100 µm and the window with the pressure transmitting membrane is arranged at a front face, as disclosed in US 8,142,362 B2, or in the wall of the housing, as disclosed in US 8,573,062 B2. Document US 8,209,031 B1 describes an implantable lead includes a lead body, having a distal end and a proximal end, configured to be implanted in a patient, and a connector provided at the proximal end.

Documents US 4,407,296 A and DE 10 2011 054 732 A1 describe hermetically sealed pressure transducers suitable for implantation in a human body.

It is an object of the invention to provide an implantable pressure sensor device which is predominantly insensitive to temperature changes in its operating environment.

An implantable pressure sensor device according to claim 1 is provided.

In one embodiment of the invention, the interior volume is the entire volume of the housing and the surface area is the entire area of the surface of the pressure transmitting membrane.

Favorably, at least 5% of the housing is made of the pressure transmitting membrane. In one embodiment of the invention, the entire housing is made of the pressure transmitting membrane.

The housing encloses an interior volume, wherein a microelectromechanical pressure sensor chip is arranged in the interior volume or coupled to the interior volume.

Favorably, the overall flexibility of the membrane is increased so that the measurement resolution is improved. As a result, the pressure load on the membrane generated by thermally initiated volume increase of the liquid inside the housing can be decreased, thus improving the measurement accuracy of the pressure measurement. In particular the housing of the implantable pressure sensor device may have a volume of not more than 100 mm³, preferably not more than 60 mm³. In particular the housing of the implantable pressure sensor device may have a diameter of not more than 10 mm, preferably of not more than 6 mm.

The interior volume comprises an incompressible liquid, such as oil, as a medium for transmitting pressure from outside the housing to the inside of the housing and the pressure sensor located therein. The flexible membrane compensates changes of the volume of the liquid in the housing caused by temperature variations during operation and manufacture without creating high pressure levels in the incompressible liquid. High pressure levels can cause nonreversible or nonlinear deformations of the membrane or damage the embedded sensor and thus, may falsify the pressure reading. By using the housing or at least a significant portion of the housing as membrane instead of arranging a membrane in a window in the housing with limited size, the surface of the membrane can be increased drastically. As a result, its capabilities for compensating volume expansions of the incompressible liquid will be improved. In particular, the overall effect of a pressure increase inside the housing is reduced. The system capacity of compensating internal volume expansions may be increased so that despite a miniaturization of the pressure sensor device below a diameter of 5 mm the temperature coefficient is not more than 10 mbar/K. The temperature coefficient describes the pressure increase inside the incompressible liquid, as a function of its temperature.

A convenient membrane material is titanium which has biocompatible and long-term stability characteristics and may be produced as hermetically tight membrane as well as sealed in a hermetically tight manner. However, the elastic modulus of titanium is high which creates a challenging problem when a miniaturized membrane is required for housing diameters of less than 5 mm, as is required for, e.g., cardiac implants. In particular for the case that a pressure level of an external volume should become transferred to an internal volume whereas both volumes are separated by a membrane, the membrane will counteract against the external load and dampen the transfer of the pressure level.

According to the invention, the membrane comprises one or more regions which allow for reversible deformation or back-formation while maintaining a size of the membrane's surface area when a volume is changed inside the housing. The membrane is at least a reasonable portion of the housing. By avoiding overall tensile stress in the membrane material, the effect of generating pressure inside the housing volume can be reduced. In particular, the geometry of the housing may feature an irregular geometry, in particular a geometry without rotational symmetry, such as a housing having an elliptical cross section, a triangular cross section, a rectangular cross section or the like. Upon volume increase inside the housing the cross section changes from the irregular cross section to a more regular cross section, e.g. changing from a cross section having no rotational symmetry to a cross section having rotational symmetry or nearly rotational symmetry. In contrast to normal stress which is applied to a longitudinal axis of the membrane which results in normal strain and material expansion, the deformation described above is dominated by bending effects where the load is applied perpendicular to a longitudinal axis of the membrane. Here, regions of tensile stress become compensated by regions of compressive stress. As a result, the overall tensile stress in the membrane vanishes as well as longitudinal expansion of the membrane material.

For instance, the housing's geometry and/or topology may be configured to generate a temperature coefficient ΔP/ΔT of not more than 10 mbar/K. Favorably, the temperature coefficient may be reduced to 5 mbar/K, being more than two orders of magnitude below the conventional arrangement with membrane covered windows in the housing.

Due to the low temperature coefficient of the housing, various tolerances become less important on the pressure sensing capabilities of the sensor.

For instance, manufacturing tolerances which may increase the oil volume only have an insignificant effect on the temperature coefficient. The functional dependency of the pressure versus volume change shows a linear behavior in a significantly larger range as when being compared to a classical front face mounted membrane window which has a rather cubic dependency (ΔVₒᵢₗ(ΔT))³. Tolerances which reduce the active membrane area, e.g. such as weld seams, interfere to a lesser extent as the ratio of membrane area to the rim area is much larger for the inventive arrangement. Plastic deformations in the housing during manufacturing such as dents, kinks and the like may in some cases be favorable for the temperature coefficient as these defects are additional irregular regions which allow for reversible deformation and back-formation upon internal volume change in the housing.

The inventive pressure sensor device is tolerant against high volume increase during thermal sterilization procedures. Typical temperatures for sterilization procedures are, for instance, about 55°C, which is about 20 K higher than the body temperature. Von-Mises tensions inside the membrane material remain in an elastic range such that the housing is not plastically deformed during sterilization. Therefore, a recalibration procedure of the pressure sensor after sterilization would become unnecessary.

Tolerances resulting from air bubbles introduced during the filling procedure when pressure transmitting liquid is filled into the housing do not dampen the pressure transmission as the membrane can compensate for the effect of air bubbles.

According to an advantageous embodiment of the invention, the housing may be configured as a tube with an elliptical cross section. The elliptical cross section may be deformed towards a circular cross section when the volume inside the housing is increased. Favorably, the tube may predominantly or completely consist of the membrane. As the elliptical cross section may change to a circular cross section when the internal volume is increased, the strain on the membrane remains low.

According to an advantageous embodiment of the invention, the sensor chip may be surrounded by the pressure transmitting membrane. Therefore the overall size of the sensor device becomes further minimized while maintaining a maximum size of the membrane surface.

In case of a conventional arrangement providing a housing with a window covered by a membrane at a front face of the housing, the pressure ΔP_{membrane} of a conventional titanium membrane scales according to the relation ΔP_{membrane} ∼ (ΔVₒᵢₗ(ΔT))³ · h · E / F⁵, according to membrane theory from continuum mechanics which only takes into account mechanical tensile stress inside the membrane, with h being membrane thickness, E being elastic modulus, and F being the membrane area. The temperature coefficient ΔP/ΔT for this arrangement is large and the temperature induced pressure measurement error becomes so large that it cannot be compensated by deduction of temperature effects. A predominant factor for the large temperature coefficient ΔP/ΔT is the tensile stress in the membrane induced by the internal volume increase inside the housing.

Typical embodiments of miniaturized pressure senor devices have liquid volume of about 25 mm³ which requires large membrane diameters of more than 5 mm when ΔP/ΔT shall be not more than 10 mbar/K.

In case of a housing consisting of a tube with an elliptical cross section, the pressure change ΔP_{membrane} of the membrane scales according to the relation ΔP_{membrane}∼(ΔVₒᵢₗ(ΔT))³ · h³ · E / F³, with h being membrane thickness, E being elastic modulus, and F being the membrane area, according to plate theory from continuum mechanics which only takes into account forces perpendicular to the sheet. The temperature coefficient ΔP/ΔT for this arrangement is much smaller than in the conventional arrangement, by up to two orders of magnitude, so that a temperature coefficient ΔP/ΔT of below 10 mbar/K can be achieved reliably even for housing diameters below 5 mm.

According to an advantageous embodiment of the invention, a front face of the housing may be provided with an opening for introducing a liquid into the housing. Oil can be introduced into the housing which can be easily sealed afterwards. In an embodiment of the invention, the opening is provided for generating an electrical connection from the pressure sensor chip within the housing with the outside of the housing.

According to an advantageous embodiment of the invention, a thickness of the membrane is not more than 30 µm, preferably not more than 25 µm. This provides sufficient flexibility and at the same time stability for the housing of the pressure sensor device.

According to an advantageous embodiment of the invention, the membrane may comprise a corrugated inner surface. The corrugated surface may be used to generate anisotropic flexural properties of the membrane.

According to an advantageous embodiment of the invention, the housing may be a tube having a longitudinal axis. Favorably, the corrugated inner surface of the membrane may comprise valleys and ridges parallel to the longitudinal axis.

Advantageously, the flexural properties of the housing may be improved. Flexure parallel to the valleys and ridges is facilitated compared to flexure perpendicular to the valleys and ridges. The material is soft when flexed parallel to the structure and stiff when flexed perpendicular to the valleys and ridges. The stability of the housing and, thus, the membrane can be improved which makes handling and transport of the pressure sensor device less critical.

According to an advantageous embodiment of the invention, an inner housing may be arranged in the housing, which inner housing encloses the pressure sensor chip. Favorably, an interior volume of the inner housing is in fluid connection to the outside of the inner housing. Within the inner housing the pressure sensor chip may be securely arranged in the housing and protected against mechanical damage.

According to an advantageous embodiment of the invention, the inner housing may be a tube having elliptical plates arranged at its opposing front faces. Favorably, the membrane may be connected to the elliptical plates.

According to an advantageous embodiment of the invention, the inner housing may feature one or more channels, where a guide wire may thread in and through the housing.

Generally, compensating volume changes by deforming the housing may be applicable in other areas too, for instance volume changes caused by chemical processes in Li-ion batteries.

The present invention together with the above-mentioned and other objects and advantages may best be understood from the following detailed description of the embodiments, but not restricted to the embodiments, wherein is shown in:
- Fig. 1: a cut view of a pressure sensor device according to an embodiment of the invention having an elliptical cross section seen from the major axis;
- Fig. 2: a cut view of the pressure sensor device of Figure 1 seen from the minor axis;
- Fig. 3: a top view of an elliptical plate of the pressure sensor device of Figure 1;
- Fig. 4: a part of a membrane tube having a corrugated inner surface;
- Fig. 5: a chart depicting the pressure-volume relationships of membrane tubes with various cross sections compared to known front face mounted membranes.

In the drawings, like elements are referred to with equal reference numerals. The drawings are merely schematic representations, not intended to portray specific parameters of the invention. Moreover, the drawings are intended to depict only typical embodiments of the invention and therefore should not be considered as limiting the scope of the invention.

The dimensions and values given in the description are intended as examples only and may be different in other embodiments of the invention.

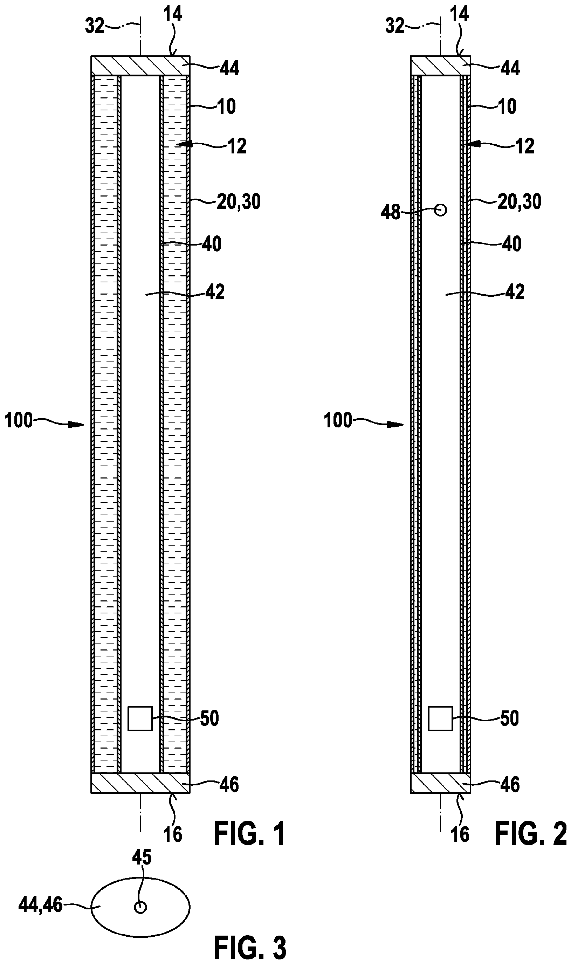

Figures 1 to 3 depict schematically an example embodiment of the invention in a cut view and a top view. A pressure sensor device 100 comprises a housing 10 enclosing an inner volume 12. The inner volume 12 is filled with an incompressible liquid such as oil. The pressure sensor device 100 is configured as a miniaturized device suitable for introducing in a human or animal body. A typical volume of such a miniaturized pressure sensor device 100 may be in the range of about 50 mm³.

The housing 10 comprises of a tube 30 closed by two opposing front faces 14, 16. Inside the housing 10 an inner housing 40 is arranged which encloses a MEMS pressure sensor chip 50 depicted in a purely schematic manner. Components such as electric cabling, electronic equipment and the like may be arranged in the inner housing 40 but are not shown. Such components are well known to a person skilled in the art.

The tube 30 of the housing 10 consists of a membrane 20, in particular made of titanium, having a thickness of not more than 30 µm, preferably not more than 25 µm.

The inner housing 40 is a massive tube with, e.g., a diameter of 1.2 mm, a length of 20 mm and a wall thickness of 100 µm. The inner housing comprises an opening 48 to establish a fluid connection between the MEMS pressure sensor chip 50 and the membrane 20.

The housing 10 and, hence, the membrane 20 have an elliptical cross section as can be seen by comparison of Figures 1 and 2. Such an irregular shape of the membrane 20 avoids tensile stress on the membrane 20 when the volume of the liquid increases with increasing temperature. The membrane 20 can compensate for the volume increase ΔVₒᵢₗ(ΔT) by deforming in direction to a regular shape, i.e. by changing from an elliptic cross section to a circular cross section. A cylinder with circular cross section has a larger cross section and, thus, a larger volume.

Elliptic plates 44, 46, in particular made of titanium, are connected, e.g. welded, to the front faces of the inner housing 40. The minor axis of the plates 44, 46 are larger than the diameter of the inner housing 40. The minor axis and the major axis may be 0.7 mm and 1.3 mm, respectively. The thickness of the plates 44, 46 may be 0.5 mm.

An opening 45 is arranged in one of the plates 44, 46 providing an inlet for filling the inner volume 12 with liquid. In an arrangement where the pressure sensor device 100 comprises electrical cables, the opening 45 may be at the distal side. In this case, the proximal front face may comprise feedthroughs for power supply of the electronic components in the pressure sensor device 100.

The membrane 20, having a thickness of e.g. 10 µm, is welded to the plates 44, 46 attached to the inner housing 40. The membrane area is about 120 mm² with dimensions of the housing 10 of, e.g., major axis 1.3 mm, minor axis 0.7 mm and height 20 mm.

The oil volume in the housing 10 is about 50 mm³, and 10 mm³ for the inner housing 40 and components therein. A temperature difference of 10 K results in a volume change of ΔVₒᵢₗ(10 K) ∼ 0.5 mm³.

This number is much lower than in a conventional arrangement with a membrane arranged over a window on a front face of a cylindrical housing.

A typical temperature gradient in the animal or human body is ΔT ∼ 1 K which leads to an approximate temperature dependent volume change ΔVₒᵢₗ(ΔT), e.g. for oil as liquid, of ΔVₒᵢₗ(ΔT) = γₒᵢₗ · Vₒᵢₗ ∼ 0.001 · Vₒᵢₗ, with γₒᵢₗ being the expansion coefficient of oil between 25°C and 100°C. Assuming the accuracy of temperature measurement being about 0.1 K, it is desirous to have a temperature coefficient ΔP/ΔT of the membrane of not more than 10 mbar/K in order to determine the pressure with an accuracy of about 2 mbar. The membrane has to be flexible enough that the resulting pressure increase generated by the volume change ΔVₒᵢₗ is less than 10 mbar.

In case of a conventional arrangement providing a housing with a window covered by a membrane at a front face of the housing, the pressure ΔP_{membrane} of a conventional titanium membrane scales according to the relation ΔP_{membrane} ∼ (ΔVₒᵢₗ(ΔT))³ · h · E / F⁵, according to membrane theory from continuum mechanics which only takes into account mechanical tensile stress, with h being membrane thickness, E being elastic modulus, and F being the membrane area. The temperature coefficient ΔP/ΔT for this arrangement is large and the temperature induced pressure measurement error becomes so large that it cannot be compensated by deduction of temperature effects. A predominant factor for the large temperature coefficient ΔP/ΔT is the tensile stress in the membrane induced by the internal pressure increase inside the housing.

In case of a housing consisting of a tube with an elliptical cross section, the pressure change ΔP_{membrane} of the membrane scales according to the relation ΔP_{membrane} ∼ (ΔVₒᵢₗ(ΔT))³ · h³ · E / F³, with h being membrane thickness, E being elastic modulus, and F being the membrane area, according to plate theory from continuum mechanics. The temperature coefficient ΔP/ΔT for this arrangement is much smaller than in the conventional arrangement, by up to two orders of magnitude, so that a temperature coefficient ΔP/ΔT of well below 10 mbar/K can be achieved reliably even for housing diameters below 5 mm.

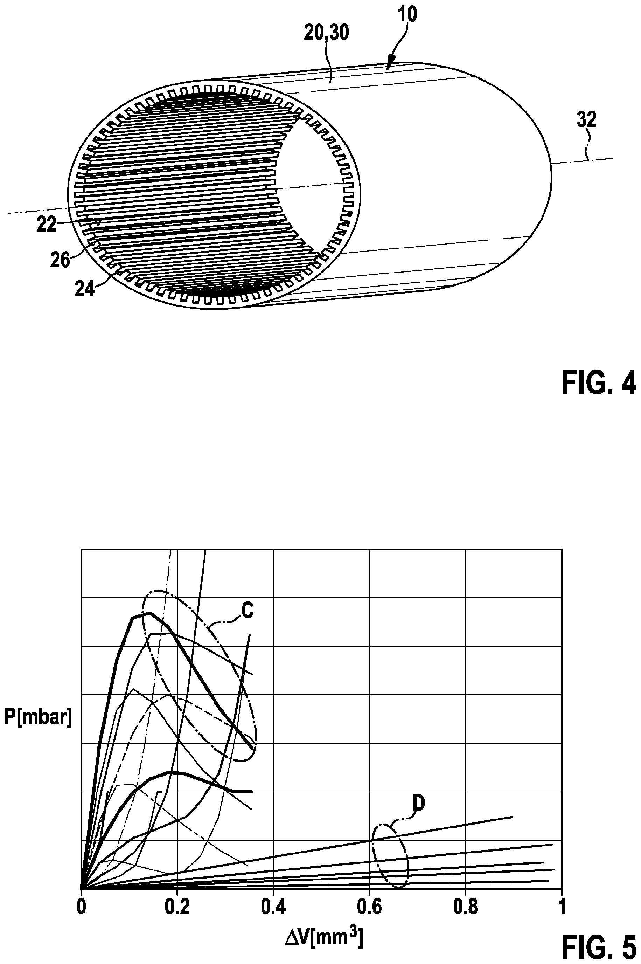

Figure 4 illustrates an example embodiment for a membrane 20 formed as a tube 30 which has a corrugated inner surface 22 with valleys 24 and ridges 26 parallel to a longitudinal axis 32 of the tube 30. The membrane thickness, i.e. thickness of the ridges 26, may be 25 µm and the thickness of the valleys 24 may be 10 µm. The stability of the membrane 20 concerning external effects during transport and implant process can be improved as thicker membranes 20 can be used.

Figure 5 illustrates simulation results of pressure-volume relationships of membrane tubes with various cross sections compared to known front face mounted membranes. A family of curves generally referred to as C represents the behavior of known front face mounted membranes exhibiting a very steep dependency of the pressure p depending on a thermally induced volume increase ΔV.

The family of curves generally denoted by D represents the behavior of tubular membranes having various cross sections such as circular, elliptical, square, triangular and the like. The curves show a nearly linear behavior of the pressure p depending on the volume increase ΔV and show only a minor dependency on the kind of the cross section.

## Claims

1. An implantable pressure sensor device (100), having a housing (10) with an interior volume (12), wherein the interior volume (12) comprises an incompressible liquid for transmitting pressure from outside the housing (10) to the inside of the housing (10), wherein a microelectromechanical pressure sensor chip (50) is arranged in the interior volume (12) or coupled to the interior volume (12), wherein the housing (10) is at least partially made of a pressure transmitting membrane (20) having a surface area,
**characterized in that**
the housing (10) comprises one or more regions which allow for reversible deformation or back-formation such that tensile stress in the membrane (20) is avoided by maintaining the size of the membrane's (20) surface area when the interior volume (12) is changed.

2. The device according to claim 1, wherein at least 5% of the housing (10) is made of the pressure transmitting membrane (20).

3. The device according to any one of the preceding claims, wherein the membrane (20) is configured to generate a temperature coefficient of not more than 10 mbar/K.

4. The device according to any one of the preceding claims, wherein the housing (10) is configured as a tube (30) with an elliptical cross section.

5. The device according to any one of the preceding claims, wherein the pressure sensor chip (50) is surrounded by the pressure transmitting membrane (20).

6. The device according to any one of the preceding claims, wherein a front face (14, 16) of the housing (10) is provided with an opening (48) for introducing the liquid into the housing (10).

7. The device according to any one of the preceding claims, wherein a thickness of the membrane (20) is not more than 30 µm, preferably not more than 25 µm.

8. The device according to any one of the preceding claims, wherein the membrane (20) comprises a corrugated inner surface (22).

9. The device according to any one of the preceding claims, wherein the housing (10) is a tube (30) having a longitudinal axis (32).

10. The device according to claim 8 and 9, wherein the corrugated inner surface (22) of the membrane (20) comprises valleys (24) and ridges (26) parallel to the longitudinal axis (32).

11. The device according to any one of the preceding claims, wherein an inner housing (40) is arranged in the housing (10), which inner housing (40) encloses the pressure sensor chip (50).

12. The device according to claim 11, wherein an interior volume (42) of the inner housing (40) is in fluid connection to the outside of the inner housing (40).

13. The device according to claim 11 or 12, wherein the inner housing (40) is a tube (30) having elliptical plates (44, 46) arranged at its opposing front faces.

14. The device according to claim 13, wherein the membrane (20) is connected to the elliptical plates (44, 46).

## Patentansprüche

1. Implantierbare Drucksensorvorrichtung (100), die ein Gehäuse (10) mit einem Innenvolumen (12) hat, wobei das Innenvolumen (12) eine inkompressible Flüssigkeit zur Übertragung von Druck von außerhalb des Gehäuses (10) zum Inneren des Gehäuses (10) aufweist, wobei ein mikroelektromechanischer Drucksensorchip (50) im Innenvolumen (12) angeordnet oder mit dem Innenvolumen (12) gekoppelt ist, wobei das Gehäuse (10) wenigstens teilweise aus einer Druckübertragungsmembran (20) hergestellt ist, die eine Mantelfläche hat,
**dadurch gekennzeichnet, dass**
das Gehäuse (10) eine oder mehr Regionen aufweist, die eine umkehrbare Verformung oder Zurückbildung ermöglichen, so dass Zugspannung in der Membran (20) vermieden wird, indem die Größe der Mantelfläche der Membran (20) aufrechterhalten wird, wenn das Innenvolumen (12) geändert wird.

2. Vorrichtung nach Anspruch 1, wobei wenigstens 5% des Gehäuses (10) aus der Druckübertragungsmembran (20) hergestellt sind.

3. Vorrichtung nach einem der vorhergehenden Ansprüche, wobei die Membran (20) zur Erzeugung eines Temperaturkoeffizienten von höchstens 10 mbar/K gestaltet ist.

4. Vorrichtung nach einem der vorhergehenden Ansprüche, wobei das Gehäuse (10) als eine Röhre (30) mit einem elliptischen Querschnitt gestaltet ist.

5. Vorrichtung nach einem der vorhergehenden Ansprüche, wobei der Drucksensorchip (50) von der Druckübertragungsmembran (20) umgeben ist.

6. Vorrichtung nach einem der vorhergehenden Ansprüche, wobei eine Vorderseite (14, 16) des Gehäuses (10) mit einer Öffnung (48) zum Einführen der Flüssigkeit in das Gehäuse (10) versehen ist.

7. Vorrichtung nach einem der vorhergehenden Ansprüche, wobei eine Dicke der Membran (20) höchstens 30 µm, vorzugsweise höchstens 25 µm ist.

8. Vorrichtung nach einem der vorhergehenden Ansprüche, wobei die Membran eine gewellte Innenfläche (22) aufweist.

9. Vorrichtung nach einem der vorhergehenden Ansprüche, wobei das Gehäuse (10) eine Röhre (30) mit einer Längsachse (32) ist.

10. Vorrichtung nach Anspruch 8 und 9, wobei die gewellte Innenfläche (22) der Membran (20) parallel zur Längsachse (32) Kehlen (24) und Grate (26) aufweist.

11. Vorrichtung nach einem der vorhergehenden Ansprüche, wobei in dem Gehäuse (10) ein inneres Gehäuse (40) angeordnet ist, wobei dieses innere Gehäuse (40) den Drucksensorchip (50) umschließt.

12. Vorrichtung nach Anspruch 11, wobei ein Innenvolumen (42) des inneren Gehäuses (40) mit dem Äußeren des inneren Gehäuses (40) in Fluidverbindung ist.

13. Vorrichtung nach Anspruch 11 oder 12, wobei das innere Gehäuse (40) eine Röhre (30) ist, die an ihren entgegengesetzten Vorderseiten angeordnete elliptische Platten (44, 46) hat.

14. Vorrichtung nach Anspruch 13, wobei die Membran (20) mit den elliptischen Platten (44, 46) verbunden ist.

## Revendications

1. Dispositif capteur de pression implantable (100) ayant un boîtier (10) avec un volume intérieur (12), le volume intérieur (12) comprenant un liquide incompressible pour la transmission de la pression de l'extérieur du boîtier (10) vers l'intérieur du boîtier (10), où une micro puce de capteur de pression (50) électromécanique est agencée dans le volume intérieur (12) ou est couplée au volume intérieur (12), où le boîtier (10) est au moins partiellement fabriqué avec une membrane (20) transmettant une pression ayant une aire de surface,
**caractérisé en ce que**
le boîtier (10) comprend une ou plusieurs régions qui permettent une déformation réversible ou une restauration à l'identique de sorte que cette contrainte de traction dans la membrane (20) est évitée en conservant la taille de l'aire de surface de la membrane lorsque le volume intérieur (12) est modifié.

2. Dispositif selon la revendication 1, dans lequel au moins 5% du boîtier (10) sont faits par la membrane (20) transmettant la pression.

3. Dispositif selon l'une quelconque des revendications précédentes, dans lequel la membrane (20) est configurée pour générer un coefficient de température de pas plus de 10 mbar/K.

4. Dispositif selon l'une quelconque des revendications précédentes, dans lequel le boîtier (10) est configuré sous forme d'un tube (30) avec une section transversale elliptique.

5. Dispositif selon l'une quelconque des revendications précédentes, dans lequel la puce de capteur de pression (50) est entourée par la membrane (20) transmettant la pression.

6. Dispositif selon l'une quelconque des revendications précédentes, dans lequel une face avant (14, 16) du boîtier (10) est pourvue d'un orifice (48) pour l'introduction du liquide dans le boîtier (10).

7. Dispositif selon l'une quelconque des revendications précédentes, dans lequel une épaisseur de la membrane (20) n'est pas supérieure à 30 µm, de préférence, n'est pas supérieure à 25 µm.

8. Dispositif selon l'une quelconque des revendications précédentes, dans lequel la membrane (20) comprend une surface intérieure (22) ondulée.

9. Dispositif selon l'une quelconque des revendications précédentes, dans lequel le boîtier (10) est un tube (30) ayant un axe longitudinal (32).

10. Dispositif selon la revendication 8 ou la revendication 9, dans lequel la surface intérieure (22) ondulée de la membrane (20) comprend des creux (24) et des arêtes (26) parallèles à l'axe longitudinal (32).

11. Dispositif selon l'une quelconque des revendications précédentes, dans lequel un boîtier interne (40) est disposé dans le boîtier (10), lequel boîtier interne (40) renferme la puce de capteur de pression (50).

12. Dispositif selon la revendication 11, dans lequel un volume intérieur (42) du boîtier interne (40) est en connexion fluidique vers l'extérieur du boîtier interne (40).

13. Dispositif selon la revendication 11 ou la revendication 12, dans lequel le boîtier interne (40) est un tube (30) ayant des plaques (44, 46) elliptiques agencées au niveau de ses faces avants opposées.

14. Dispositif selon la revendication 13, dans lequel la membrane (20) est connectée aux plaques (44, 46) elliptiques.
